# EUROPÄISCHE PATENTSCHRIFT

(11) **EP 2 590 659 B1**
(45) Veröffentlichungstag und Bekanntmachung des Hinweises auf die Patenterteilung: **08.11.2017**
(21) Anmeldenummer: 11764081.3
(22) Anmeldetag: 21.06.2011
(51) Int. Cl.: A61K 35/12, A61K 35/14, A61K 38/17, A61P 19/02

(54) **GELSOLIN-ANGEREICHERTES BLUT ZUR THERAPEUTISCHEN VERWENDUNG**
GELSOLIN-ENRICHED BLOOD FOR THERAPEUTIC USE
SANG ENRICHI DE LA GELSOLINE POUR UNE UTILISATION THERAPEUTIQUE

(30) Priorität: 07.07.2010 DE 102010026500
(43) Veröffentlichungstag der Anmeldung: 15.05.2013
(73) Patentinhaber: Schneider, Ulrich, 83707 Ringsee (DE); Arthrogen GmbH, 83707 Bad Wiessee-Ringsee (DE)
(72) Erfinder: Schneider, Ulrich., 83707 Ringsee (DE)
(74) Vertreter: Rudolph, Ulrike
(86) Internationale Anmeldenummer: PCT/DE2011/001322
(87) Internationale Veröffentlichungsnummer: WO 2012/010128

(56) Entgegenhaltungen:
- WO-A1-99/09051
- US-A1- 2003 108 612
- DOBROVOLSKAIA M A ET AL: "Interaction of colloidal gold nanoparticles with human blood: effects on particle size and analysis of plasma protein binding profiles", NANOMEDICINE: NANOTECHNOLOGY, BIOLOGY AND MEDICINE, ELSEVIER, NL, Bd. 5, Nr. 2, 1. Juni 2009 (2009-06-01), Seiten 106-117, XP026150653, ISSN: 1549-9634, DOI: 10.1016/J.NANO.2008.08.001 [gefunden am 2008-12-13]
- VARGAS T ET AL: "Protection by gelsolin on amyloid-b-induced toxicity in the blood-CSF-brain barrier: Apoptotic pathways", JOURNAL OF NEUROLOGICAL SCIENCES, ELSEVIER SCIENTIFIC PUBLISHING CO, AMSTERDAM, NL, Bd. 283, Nr. 1-2, 15. August 2009 (2009-08-15), Seite 299, XP026320660, ISSN: 0022-510X, DOI: 10.1016/J.JNS.2009.02.225 [gefunden am 2009-07-14]
- WIWANITKIT ET AL: "Increasing the agglutination reaction in slide test for weak B blood group by gold nanoparticle solution: The first world report", JOURNAL OF IMMUNOLOGICAL METHODS, ELSEVIER SCIENCE PUBLISHERS B.V.,AMSTERDAM, NL, Bd. 328, Nr. 1-2, 30. Oktober 2007 (2007-10-30), Seiten 201-203, XP022322512, ISSN: 0022-1759, DOI: 10.1016/J.JIM.2007.09.005
- OSBORN TERESIA M ET AL: "Decreased levels of the gelsolin plasma isoform in patients with rheumatoid arthritis", ARTHRITIS RESEARCH AND THERAPY, BIOMED CENTRAL, LONDON, GB, Bd. 10, Nr. 5, 27. September 2008 (2008-09-27), Seite R117, XP021046820, ISSN: 1478-6354, DOI: 10.1186/AR2520
- SHAW C F: "GOLD-BASED THERAPEUTIC AGENTS", CHEMICAL REVIEWS, AMERICAN CHEMICAL SOCIETY, US, Bd. 99, Nr. 9, 1. September 1999 (1999-09-01), Seiten 2589-2600, XP000852439, ISSN: 0009-2665, DOI: 10.1021/CR980431O

## Beschreibung

Offenbart wird ein Verfahren zur Herstellung mindestens eines therapeutisch wirksamen Proteins oder eines Proteingemisches in einem Behältnis, wobei das Behältnis mit einer Körperflüssigkeit gefüllt, inkubiert und das therapeutisch wirksame Protein in der Körperflüssigkeit gebildet wird. Offenbart wird außerdem ein Behältnis für die Durchführung des Verfahrens und Arzneimittel, die die derart hergestellten Proteine als Wirkstoff enthalten.

Degenerative Gelenkerkrankungen haben sowohl beim Menschen als auch bei Tieren eine große Bedeutung. Beim Menschen tritt die Arthrose idiopathisch (mit bekannten Risikofaktoren) beim älteren Patienten auf, oder kann bei jüngeren Patienten als Komplikation posttraumatisch bedingt entstehen. Beide Formen haben aber die gleichen klinischen Symptome, u.a. Gelenkschmerzen und eingeschränkte Funktion, und führen häufig zu einer stark eingeschränkten Lebensqualität des betroffenen Patienten. Verschiedene Ursachen wie Überbelastung, Fehlbelastung, Gelenkinstabilität oder auch Infektionen führen zu einer mechanischen und enzymatischen Schädigung des Gelenkknorpels mit Apoptose der Chondrozyten, sowie Verlust an Kollagen Typ II und Proteoglykanen. Dabei besteht eine Imbalance zwischen Degeneration und Reparation. Obwohl im Mittelpunkt der Pathogenese die Knorpeldegeneration steht, betrifft die Krankheit nicht nur den Knorpel, sondern auch die Gelenkskapsel und den subchondralen Knochen. Bei Knorpelschäden gelangen die Abbauprodukte in die Synovia und führen zu einer Synovitis. Zudem können auch Schäden an der Gelenkskapsel direkt zu einer Freisetzung von Entzündungsmediatoren führen und eine stark traumatisierte Gelenkskapsel resultiert zudem in einer Gelenksinstabilität. Bei hoher und zyklischer Belastung adaptiert sich die subchondrale Knochenplatte mit einer erhöhten Knochendichte. Durch diese Sklerosierung wird aber der Knochen steifer und spröder. Dies führt einerseits zu einer Abnahme der Fähigkeit zur Schockabsorption, was den darüber liegenden Knorpel mehr belastet, und andererseits zu Scherkräften am Übergang subchondrale Knochenplatte und mineralisiertem Knorpel. Bei Pferden mit Arthrose und/oder Osteoarthritis und/oder anderen Gelenkserkrankungen wurden erhöhte Konzentrationen der proinflammatorischen (entzündungsfördernden) Zytokine Tumor Nekrose Faktor alpha (TNFa), Interleukin 1 (IL-1) und Interleukin 6 (IL-6) sowie an Prostaglandin E2 (PGE2) und Metalloproteasen in der Synovia gemessen. Auch bei Menschen mit Arthrose ist die Konzentration der proinflammatorischen Zytokine im Blut und in der Synovia erhöht.

Die proinflammatorischen (entzündungsfördernden) Zytokine TNFα, IL-1 und IL-6 werden von den B-Synoviozyten (Synoviocyti secretorii) der Synovialmembran, von Entzündungszellen in der Synovialmembra und von Chondrozyten sezerniert und stimulieren die Freisetzung von Matrixmetalloproteasen (MMP's) und Aggrecanasen sowie von weiteren Entzündungsmediatoren wie Prostaglandinen (PGE2) oder Nitritoxid (NO). Metalloproteasen sind Enzyme, welche die Matrix des Knorpel (Kollagen Typ II, Proteoglykane u.a.) degradieren. II-1 und TNFα hemmen auch direkt die Produktion von Typ II Kollagen.

Die herkömmliche Therapie der degenerativen Gelenkerkrankungen besteht in den meisten Fällen in einer symptomatischen Behandlung der Entzündung, d.h. in einer entweder systemischen oder intraartikulären Hemmung der Entzündung mittels entsprechender Medikamente. Hierzu gehören die am häufigsten eingesetzten intraartikulär applizierten Kortikosteroiden, teilweise in Kombination mit Hyaluronsäure. Zusätzlich werden gelegentlich außerdem Chondroprotektiva verabreicht.

Diese medikamentösen Therapien haben jedoch zahlreiche Nebenwirkungen.

Eine Alternative zu den symptomatischen Therapien (auf der Basis nicht steroidaler und steroidaler Entzündungshemmer) bilden die Behandlungen mit Zytokin-Inhibitoren oder Chondroprotektiva, die als "disease modifying drugs" bezeichnet werden (Qvist et al. 2008). Hierzu zählen auch Therapien, bei denen körpereigene (autologe) Proteine aus dem Blut des Patienten gewonnen und als individuelles Medikament diesem Patienten wieder verabreicht werden.

Aus der WO 9909051 ist ein Verfahren bekannt zur Herstellung von therapeutisch wirksamen, autologen (= körpereigenen) Proteinen in einer zuvor vom (tierischen oder menschlichen) Patienten gewonnenen Körperflüssigkeitsprobe und eine Spritze zur Durchführung dieses Verfahrens. Als derart herstellbare Proteine sind Erythropoietin, Insulin, Interferone, Interleukin 4, Interleukin 10, löslicher Tumor-Nekrose-Faktor-Rezeptor und der Interleukin-1 Rezeptor-Antagonist genannt. Die Proteine werden in einer Spritze hergestellt und bereitgestellt.

Die inneren Strukturen der Spritze, wozu auch in der Spritze angeordnete Partikel, insbesondere mit Chromsulfat imprägnierten Glaskügelchen, zählen, sind mit immobilisierten Induktoren beschichtet, die die Biosynthese der gewünschten Proteine anregen sollen. Im Fall von Blut als Körperflüssigkeitsprobe sind als solche Induktoren Immunglobuline, insbesondere Immunglobulin G vorgesehen, um die im Blut enthaltenen Monocyten zur Bildung anti-inflammatorischer Proteine anzuregen.

Für die Durchführung des Verfahrens wird die Spritze mit einer Körperflüssigkeit eines Patienten gefüllt und inkubiert. Dabei wird das therapeutisch wirksame Protein in der Körperflüssigkeit gebildet.

Die so angereicherte Körperflüssigkeit kann in der Spritze steril gelagert und bei Bedarf dem Patienten direkt ohne weitere Behandlung oder beispielsweise nach Zentrifugation und/oder Sterilfiltration wieder zugeführt werden. Ein Aspekt der vorliegenden Offenbarung besteht darin, ein solches Verfahren derart weiterzuentwickeln bzw. abzuwandeln, dass die erzeugten Proteine in signifikant höheren Mengen vorliegen, und dass unerwünschte Begleiterscheinungen vermieden sind. Die Offenbarung zeigt die Bereitstellung eines Verfahrens der eingangs genannten Art, bei dem das Behältnis Goldpartikel enthält.

Der Begriff 'Behältnis' steht im Folgenden für ein verschließbares Gefäß oder einen verschließbaren Behälter zur Aufbewahrung von Flüssigkeiten für eine bestimmte Zeit, wobei Gefäß bzw. Behälter dicht gegenüber der aufzunehmenden Flüssigkeit ist.

Der Einsatz von Gold als Arzneimittel ist in der Medizin schon lange bekannt. Ende des 19. Jahrhunderts wurde Gold vor allem als Arzneimittel zur Behandlung von Tuberkulose eingesetzt. Aufgrund der falschen Annahme, dass die rheumatoide Arthritis ebenfalls eine Infektionserkrankung sei, wendete man Gold als Arzneimittel auch bei diesem Leiden an. Die Therapie war erfolgreich und wurde dann über viele Jahre bis in die heutige Zeit angewendet (Kean and Kean 2008). Eine in vitro Studie an humanen Chondrozyten hat gezeigt, dass Goldverbindungen (Aurothiomalat) die Produktion von Nitrit Oxid (NO) der Chondrozyten hemmt. Nitrit Oxid vermittelt (mediiert) die destruktiven Effekte der proinflammatorischen Zytokine IL-1 und TNFα (Green et al. 2006). Dies führt zu einer verminderten Kollagen- und Proteoglykanproduktion, zur Chondrozytenapoptose und zu einer Stimulation der Metalloproteasen (Vuolteenaho et al. 2005).

In den im Stand der Technik bekannten therapeutischen Ansätzen wird das Gold entweder intramuskulär oder per os appliziert. Mit diesen Applikationsmethoden und der entsprechenden Galenik von Gold werden die aus den in vitro-Studien bekannten gewünschten Effekte jedoch nur ansatzweise erreicht.

Die in dem offenbarungsgemässen Verfahren erstmals eingesetzte Kombination von Goldpartikeln mit einer menschlichen oder tierischen autologen oder homologen Körperflüssigkeit, insbesondere einer Eigenblutprobe, in einem abgeschlossenen System liefert überraschenderweise nicht nur (i) eine im Vergleich zu dem vorbekannten Verfahren deutlich höhere Konzentration der gewünschten Proteine, im Fall von Blut insbesondere der Zytokine (vor allem IL-1, IL-6, IL-8, IL-10, G-CSF, MCP-1, MIP-1, RANTES, TNF-alpha, GRO-alpha, MCP-3, MIF und IL-1RA), sondern (ii) auch eine bessere Qualität der betreffenden Proteine aufgrund einer Hemmung der physiologische Proteinalterung während der Inkubationszeit und (iii) außerdem eine signifikante Anreicherung des Proteins Gelsolin. Gelsolin ist ein ubiquitär in allen tierischen (inklusive menschlichen) Zellen und auch extrazellulär (z.B. im Blutplasma) vorhandenes Aktinbindendes Protein, das Ca²⁺-abhängig Aktinfilamente fragmentiert und eine Repolymerisation verhindert, und das eine Schlüsselfunktion bei der Regulation der Aktinfilament-Aufbau und -Abbauprozesse hat/erfüllt. Gelsolin wurde in zytoplasmatischen Extrakten entdeckt und identifiziert, und seine ubiquitäre Präsenz und phylogenetische Konservierung in freibeweglichen Zellen spricht für seine essentielle Rolle als ein intrazelluläres regulatorisches Protein. Erst später wurde entdeckt, dass Gelsolin auch im Blutplasma von Säugern vorkommt und dort Aktin depolymerisiert. Dieses sogenannte Plasmagelsolin (pGelsolin, pGS) ist eine Isoform des zytoplasmatischen Gelsolins (cGelsolin, cGS) und unterscheidet sich von diesem strukturell dadurch, dass es am N-terminalen Ende zusätzliche 23 Aminosäuren aufweist. Die physiologische Funktion von PlasmaGelsolin ist nach wie vor Gegenstand zahlreicher Forschungsarbeiten (DiNubriu, 2007 und darin zitierte Literatur). Gelsolin reguliert so wichtige Zellfunktionen wie Zellmotilität, Phagozytose, Apoptose und Aktivierung von Thrombozyten (Silacci et al. 2004, Trickey et al. 2004). Bei Menschen mit rheumatoider Arthritis ist die Plasmakonzentration von Gelsolin vermindert (Osborn et al. 2008). Auch bei anderen Erkrankungen mit Gewebedegenerationen und insbesondere bei Sepsis ist die Plasmakonzentration von Gelsolin vermindert (Suhler et al. 1997, Osborn et al. 2008, Lee et al. 2007). Im Stand der Technik vorhandene Erkenntnisse weisen darauf hin, dass PlasmaGelsolin als Puffer dient, um überschießende Entzündungsreaktionen des Körpers abzufangen (DiNubile 2008).

Die mit dem offenbarungsgemässen Verfahren hergestellten Proteine, insbesondere Gelsolin und Zytokine, können (müssen jedoch nicht) zusammen mit den anderen Bestandteilen der in dem Behältnis befindlichen Flüssigkeit dem Patienten direkt, das heißt ohne weitere Manipulation - wie z.B. Zentrifugation, Sterilfiltration oder Umfüllen in andere Behälter - wieder appliziert werden. Dadurch wird eine Kontamination der Protein-Lösung vermieden und das Risiko einer Infektion des Patienten bei der Verabreichung der Proteine wird minimiert.

Bei einer Variante des offenbarungsgemässen Verfahrens werden die Goldpartikel nach der in vitro Inkubation aus der Körperflüssigkeit, beispielsweise dem Serum entfernt und verworfen. Das hat den Vorteil, dass goldinduzierte Nebenwirkungen bei oder nach der Verabreichung dieser Körperflüssigkeit, beispielsweise dieses Serums, komplett vermieden werden.

Bei einer ebenfalls bevorzugten Variante des offenbarungsgemässen Verfahrens werden sowohl die Goldpartikel als auch Körperzellen (beispielsweise Blutzellen) und andere unlöslichen Aggregate nach der in-vitro Inkubation aus der Körperflüssigkeit (beispielsweise dem Blut) entfernt und verworfen. Derartig aufbereitete autologe humane Körperflüssigkeiten und insbesondere Seren sind hervorragend verträglich, und Nebenwirkungen sind nicht zu erwarten.

Die in dem Verfahren eingesetzten Goldpartikel weisen vorzugsweise eine definierte Struktur und/oder eine definierte Größe auf. Mikro- und/oder Nanopartikel mit einer Partikelgröße zwischen 10 Nanometern bis 500 Mikrometern sind besonders geeignet.

Bei den Varianten des offenbarungsgemässen Verfahrens, bei denen die Goldpartikel nach der in vitro Inkubation aus der Körperflüssigkeit, beispielsweise dem Serum, zu entfernen und zu verwerfen sind, werden in dem Verfahren vorzugsweise Goldpartikel mit der Größe von etwa 1 µm eingesetzt, weiter vorzugsweise in einer Menge von 10³ - 10⁴ Goldpartikel pro 10 ml. Für diese Anwendung habe sich in der Praxis Behältnisse (z.B. eine Spritze) bewährt, deren Fassungsvolumen etwa 10 ml beträgt.

In einer Ausführungsform, die sich in der praktischen Anwendung gut bewährt hat, liegen die Goldpartikel in dem Behältnis in einer Menge von 0,3 mg pro 1 ml Körperflüssigkeit vor. Konzentrationen von 0,1 bis 10 mg pro 1 ml sind grundsätzlich geeignet und vorgesehen.

Die innere Struktur des Behältnisses ist vorzugsweise frei von Antikoagulantien wie Heparin, Citrat, EDTA oder CPDA, denn im Rahmen der dieser Erfindung zugrunde liegenden Arbeiten wurde überraschenderweise gefunden, dass bei Anwesenheit solcher Substanzen weniger Proteine biosynthetisiert werden als bei Abwesenheit. Insbesondere im Fall von Blut als Körperflüssigkeit und Heparin als Antikoagulanz wurde bei Anwesenheit von Heparin, z.B. als Beschichtung der Behältnisinnenwand, eine erheblich geringere Zytokinproduktion erhalten als bei Abwesenheit von Heparin und anderen Antikoagulantien.

Als Behältnis kommt insbesondere eine Spritze in Betracht, weil diese nicht nur als Inkubationsgefäß dienen kann, sondern gleichzeitig auch als Entnahmeinstrument für die Gewinnung der Körperflüssigkeit und/oder als Applikationsinstrument für die Verabreichung der Proteine an einen Patienten geeignet ist.

Als Behältnis kommt aber auch ein verschließbarer Beutel in Betracht, weil dieser insbesondere bei größeren Volumina flexibler gehandhabt und gelagert werden kann als eine Spritze mit vergleichbarem Volumen, und weil er technisch einfach und sicher mit einer Spritze gekoppelt werden kann, so dass das Befüllen und Entleeren über diese erfolgen kann.

Das offenbarungsgemässe Verfahren eignet sich ganz besonders für die Herstellung (Biosynthese) und Anreicherung von Proteinen aus Blutzellen. Deshalb ist als Körperflüssigkeit insbesondere Blut vorgesehen.

Überraschenderweise lässt sich das therapeutisch wirksame Protein Gelsolin besonders gut, d.h. in signifikanten Mengen mit dem erfindungsgemäßen Verfahren herstellen und anreichern. Deshalb ist das offenbarungsgemässe Verfahren insbesondere zur Gewinnung von Gelsolin vorgesehen.

Hinsichtlich der Inkubationsbedingungen für das mit Körperflüssigkeit befüllte Behältnis hat sich in der Praxis gezeigt, dass ein Inkubationszeit von 12 bis 72 Stunden, bevorzugt 24 Stunden, bei einer Temperatur von 20°C bis 41 °C, vorzugsweise 37°, zu guten Ergebnissen führt.

Eine Lösung der genannten Aufgabe besteht auch in der Bereitstellung eines Behältnisses für die in-vitro Biosynthese und Anreicherung (In-vitro Induktion) von therapeutisch wirksamen Proteinen in einer Körperflüssigkeit, das sich dadurch auszeichnet, dass das Behältnis Goldpartikel enthält und zur Aufnahme, Lagerung und Wiederabgabe einer Körperflüssigkeitsprobe geeignet ist, und dass es mit einer Hohlnadel (Kanüle, Injektionsnadel) derart koppelbar ist, dass sein Inhalt vermittels dieser Hohlnadel (Kanüle, Injektionsnadel) injiziert werden kann.

Mit diesem Behältnis lassen sich insbesondere das zuvor beschriebene Protein-Herstellungsverfahren durchführen und die mit diesem einhergehenden Vorteile nutzen. Die hergestellten Proteine können mittels der ankoppelbaren Hohlnadel direkt an den gewünschten Applikationsort gebracht werden, insbesondere in einen menschlichen oder tierischen Körper eingebracht werden.

Die Goldpartikel weisen vorzugsweise eine definierte Struktur und/oder eine definierte Größe auf. Mikro- und/oder Nanopartikel (Partikelgröße zwischen 10 Nanometern und 500 Mikrometern) sind besonders geeignet. Eine geeignete Menge Goldpartikel in dem Behältnis beträgt 0,1 mg bis 10 mg pro 1 ml Körperflüssigkeit, vorzugsweise 0,3 mg pro 1 ml Körperflüssigkeit.

Bei dem Behältnis handelt es sich vorzugsweise um eine Spritze oder um einen Beutel. Die Spritze hat den Vorteil, dass sie nicht nur als Inkubationsgefäß dienen kann, sondern gleichzeitig auch als Entnahmeinstrument für die Gewinnung der Körperflüssigkeit und/oder als Applikationsinstrument für die Verabreichung der Proteine an einen Patienten. Der Beutel hat den Vorteil, dass er bei größeren Volumina flexibler gehandhabt und gelagert werden kann als eine Spritze mit vergleichbarem Volumen und für das Befüllen und Entleeren mit einer Spritze gekoppelt werden kann.

Als Behältnis eignet sich besonders gut eine handelsübliche Spritze (beispielsweise 5 bis 100 ml Spritzen) ohne besondere Ausgestaltung in ihrem inneren Hohlraum. In den Spritzenzylinder werden die Goldpartikel (z.B. Gold Microcarriers von BioRad Laboratories, Cat. 165-2264) eingebracht.

Die Spritze, insbesondere die Innenwand des Spritzenzylinders und der in dem Zylinder liegenden Teil des Spritzenkolbens besteht vorzugsweise aus einem Kunststoff, beispielsweise aus Polystyrol, Polyethylen, Polyvinylchlorid, Polypropylen (neutrale S-Monovetten, Sarstedt) oder einem ähnlichen Stoff oder Gemischen davon.

Das Behältnis eignet sich besonders gut für die Herstellung (Biosynthese) und Anreicherung von Proteinen, insbesondere von Gelsolin, aus Blutzellen. Deshalb ist als Körperflüssigkeit insbesondere Blut vorgesehen.

Die mit dem offenbarungsgemässen Verfahren in einer Körperflüssigkeit hergestellten Proteine können zusammen mit der Körperflüssigkeit und den Goldpartikeln oder ohne die Goldpartikel als Arzneimittel zur Behandlung von Krankheiten eingesetzt werden.

Die mit den offenbarungsgemässen Verfahren hergestellten proteinangereicherten und insbesondere Zytokinin- und Gelsolinangereicherten Körperflüssigkeiten, insbesondere Blutseren, stellen eine sichere, kostengünstig und schnell herzustellende und besonders nebenwirkungsarme Alternative zu vergleichbaren konventionellen Arzneimittelpräparaten dar.

Die vorliegende Erfindung betrifft ein Zytokinin- und Gelsolinangereichertes Blut, zur Verwendung als Arzneimittel bzw. zur Herstellung eines Arzneimittels, erhältlich dadurch, dass Blut, in ein Behältnis aufgenommen wird, welches Goldpartikel enthält, vorzugsweise Goldpartikel mit der Größe von etwa 1 µm und vorzugsweise in einer Menge von 10³ - 10⁴ Goldpartikel pro 10 ml Behältnis (oder 0.3 mg Goldpartikel mit einem Durchmesser 1 µm pro 1 ml Blut/Behältnis), dass diese Mischung aus Blut, und Goldpartikeln inkubiert wird (beispielsweise über 12-72 Stunden, vorzugsweise über 24 Stunden und bei 20°C bis 41 °C, vorzugsweise bei 37°C), und dass anschließend die Goldpartikel und vorzugsweise (d.h. optional) zudem Blutzellen und andere unlösliche Bestandteile aus der Körperflüssigkeit, insbesondere dem Blut, entfernt und verworfen werden (vorzugsweise durch Zentrifugation und/oder Sterilfiltration).

Die Offenbarung betrifft außerdem die Verwendung von autologem oder homologem Blut und Goldpartikeln in Kombination als Arzneimittel bzw. zur Herstellung eines Arzneimittels. Mit anderen Worten: Gegenstand vorliegender Erfindung ist auch eine Stoffmischung aus autologem oder homologem Blut und Goldpartikeln zur Verwendung als Arzneimittel, bzw. ein Arzneimittel umfassend die Stoffmischung autologes oder homologes Blut und Goldpartikeln inklusive der darin angereicherten Proteine als Wirkstoffkombination.

Die offenbarten Arzneimittel ermöglichen eine besonders einfache, kostengünstige, risikoarme, und effektive Behandlung.

Die beschriebenen Arzneimittel eignen sich insbesondere für die Behandlung von degenerativen Gewebeerkrankungen, vor allem von Arthrosen und Tendinosen. Vor allem das mit Goldpartikeln inkubierte und anschließend von Partikeln befreite Arzneimittel ist für die Behandlung von Arthrose und anderen Krankheiten, die mit Gewebedegenerationen assoziiert sind, und/oder die mit einem Gelsolinmangel assoziiert sind, gut geeignet und dafür vorgesehen.

Eine besondere Ausführungsform der erfindungsgemäßen Arzneimittel zeichnet sich dadurch aus, dass die mit Goldpartikeln inkubiertes Blut nach der Inkubation einen Gehalt an Gelsolin aufweist, der wenigstens das Doppelte des entsprechenden Blutnormwerts für Gelsolin beträgt. Der Begriff "entsprechender Blutnormwert für Gelsolin" steht im vorliegenden Kontext für den humanmedizinischen oder tiermedizinischen Normwert für Gelsolin im Blut derjenigen Patientengruppe, die mit dem Arzneimittel behandelt werden soll. Die Patientengruppe ist dabei charakterisiert durch ihre zoologische Art- und Rassenzugehörigkeit, Geschlecht und Alter.

Dieses Gelsolin-reiche Arzneimittel ist für die Behandlung von Arthrose vorgesehen, die mit einem Gelsolinmangel im Blut des Patienten einhergeht. Das inkubierte Blut-Gold-Gemisch kann als Ganzes oder in Teilen appliziert werden. Bei Bedarf kann es vor der Verabreichung an den (tierischen oder menschlichen) Patienten noch einer Zentrifugation und/oder Sterilfiltration unterworfen werden, um z.B. Zellen und Zellfragmente aus dem Blut zu entfernen und damit gleichzeitig auch das Injektionsvolumen zu verringern.

Die Erfindung wird im folgenden anhand von Ausführungsbeispielen und dazugehörigen Figuren näher erläutert:
- Figur 1 zeigt:: eine MID-FTIR-Spektroskopie-Analyse des Proteinprofils in Blutseren verschiedener Patienten vor (T0) und nach (T24) Durchführung des offenbarten Verfahrens im Vergleich zu Kontrollen. BLAU repräsentiert das Proteinprofil für den Zeitpunkt T0, GRÜN zeigt das Proteinprofil der mit dem erfindungsgemäßen Verfahren behandelten Proben zum Zeitpunkt T24, ROT zeigt das Proteinprofil der Kontrollseren zum Zeitpunkt T24.
- Figur 2 zeigt:: die Ergebnisse einer Multiplex-Analyse der Proteine:
GS = Gelsolin
IL-4 = Interleukin-4
IL-10 = Interleukin-10
IL-13 = Interleukin-13
IL-1Ra = Interleukin-1 Rezeptorantagonist
IL-1ß = Interleukin-1ß
TNF-α = Tumornekrasefaktor alpha
G-CSF = Granulocyte-Colony Stimulating factor
GM-CSF = Granulocyte-Macrophage Colony Stimulating factor
IFN-g = Interferon Gamma
SCGF-ß = Hematopoietic Stem cell growth factor
MIP-1a = Macrophage inflammatory protein-1a
MIP-1ß = Macrophage inflammatory protein-1ß
VEGF = Vascular endothelial growth factor
IL-18 = Interleukin-18
MCP-3 = Macrophage chemotactic protein
SDF-a = Stromal derived factor
basic FGF = Fibroblast growth factor
GROa = Growth-regulated oncogene alpha in Blutproben zum Zeitpunkt T0 ("T0") und nach 24 Stunden Inkubation einerseits ohne weitere Behandlung ("Kontrolle"), und anderseits mit Behandlung entweder gemäß WO 9909051 ("StdT") oder gemäß dem offenbarten Verfahren ("Erfindung").

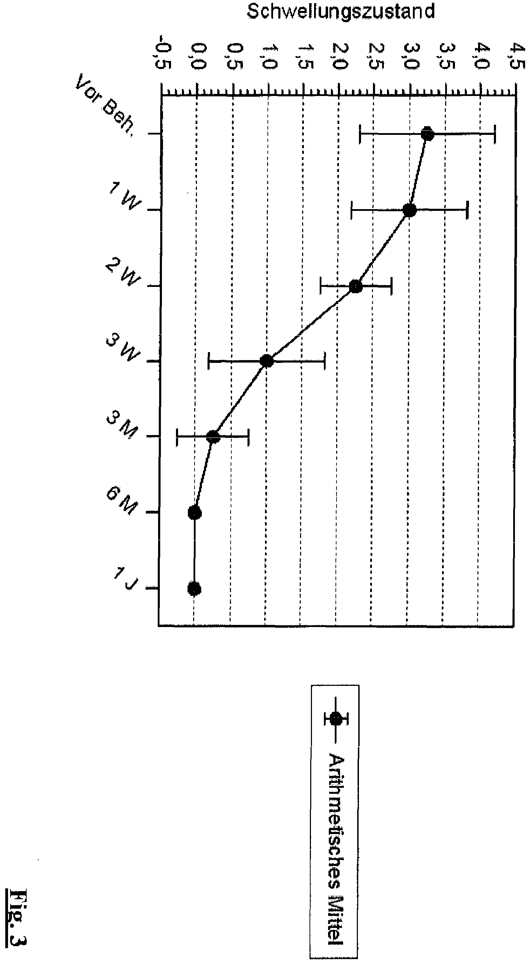

- Figur 3 zeigt:: die graphische Dokumentation des Schwellungsgrades aller untersuchten Pferde vor bzw. nach der erfindungsgemäßen Behandlung zu den jeweiligen Nachuntersuchungszeitpunkten

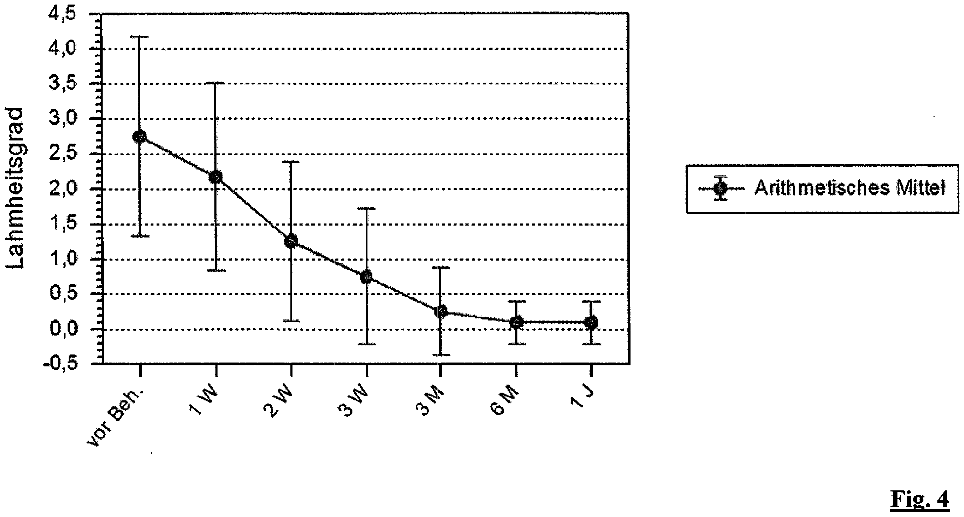

- Figur 4 zeigt:: die graphische Dokumentation der Lahmheit aller untersuchten Pferde vor bzw. nach der erfindungsgemäßen Behandlung zu den jeweiligen Nachuntersuchungszeitpunkten

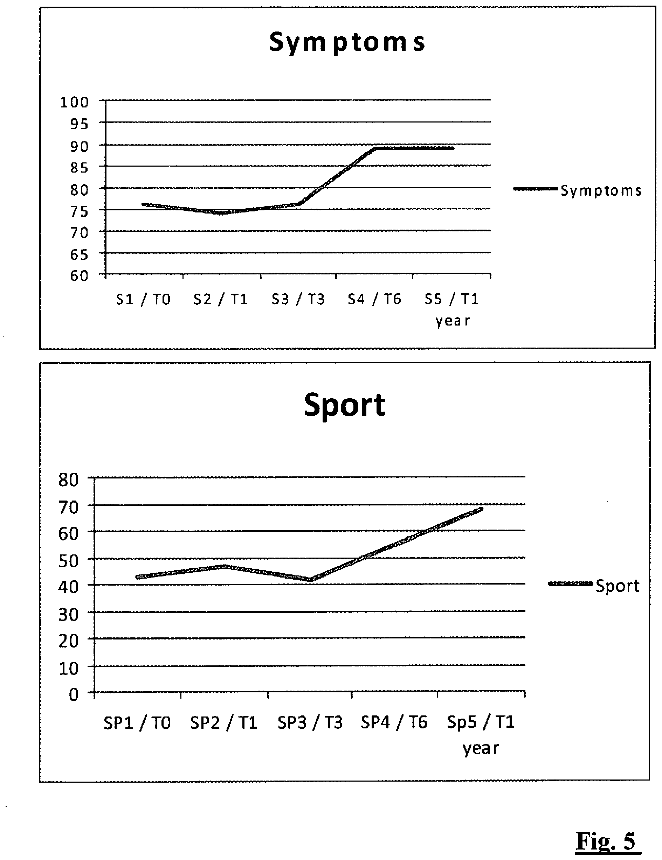

- Figur 5 zeigt:: KOOS-Score vor und nach der Behandlung bei Patienten mit Gonarthrose

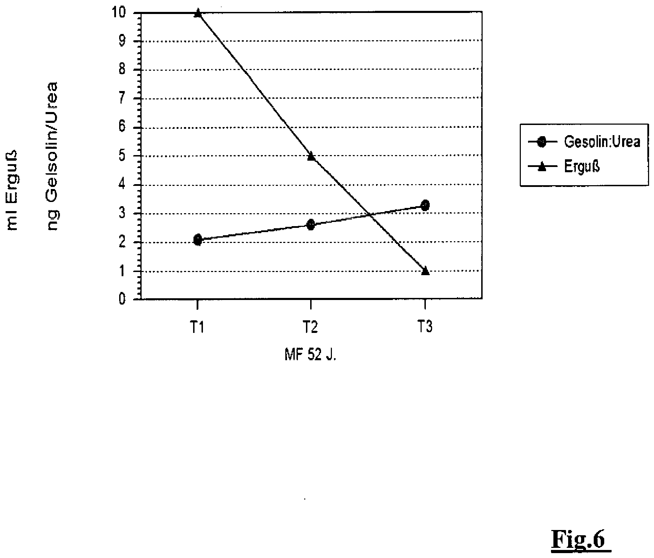

- Figur 6 zeigt:: Ergussbildung und Gelsolinkonzentration in der Synovialflüssigkeit bei einer Patientin mit Gonarthrose im Verlauf: nach der 1. (T1), 2. (T2) bzw 3. Injektion (T3)

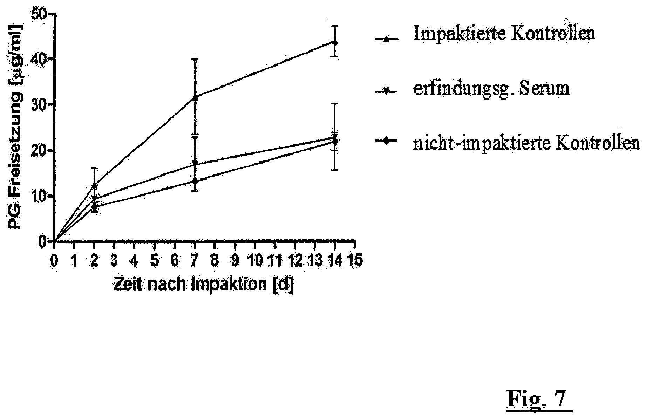

- Figur 7 zeigt:: die Proteoglykanfreisetzung von Explantaten/Knorpel-Knochen-Präparaten nach Stoßbehandlung.

### Beispiel 1: Überprüfung der Proteinherstellung in einer Blutpobe nach dem offenbarten Verfahren und in einem offenbarten Behältnis mittels MID-FTIR-Spektroskopie-Verfahren und Multiplex-Analyse

Jeweils zwei Blutproben der Menge 9 ml von 11 verschiedenen Patienten (Nr. 1-11) wurden in ein offenbartes Behältnis, nämlich eine zuvor mit 2,7 mg Goldpartikeln (mit einem Durchmesser von 1 µm) befüllte 9ml Spritze aufgenommen. Eine gleiche Menge Blutprobe desselben Ursprungs (Patienten) wurde in eine gleichartige 9ml Spritze ohne Gehalt an Goldpartikeln aufgenommen. Die Proben wurden zu unterschiedlichen Zeitpunkten analysiert, nämlich zum Zeitpunkt T0 sofort nach der Blutentnahme und zum Zeitpunkt T24 nach Inkubation für 24 Stunden bei 37°C.

Mittels Fourier-Transformations-Infrarot-Spektroskopie im mittleren Infrarot-Bereich (Spektralspektrum von 4000 cm⁻¹ bis 400 cm⁻¹), kurz MID-FTIR-Spektroskopie (z.B. dem AquaSpec-Verfahren der Firma Micro-Biolytics GmbH/Esslingen) wurden die Proben analysiert.

Das Messprinzip der Fourier-Transformations-Infrarot-Spektroskopie) beruht auf der Bestrahlung eines Stoffes mit elektromagnetischen Wellen, wobei bestimmte Frequenzbereiche absorbiert werden. Da Infrarotstrahlung energetisch im Bereich der Schwingungsniveaus von Molekülbindungen liegt, führt die Absorption zu einer Schwingungsanregung der Bindungen. Diese wird in Form von Ausschlägen im gemessenen Spektrum (Diagramm) sichtbar. Da die dazu notwendigen Energien bzw. Frequenzen charakteristisch für die jeweiligen Bindungen sind, können so auch Materialien identifiziert und Strukturen aufgeklärt werden.

Die FTIR-Spektroskopie ist insbesondere für die Analyse struktureller, reaktionsinduzierter Veränderungen in einem biologischen Makromolekül geeignet ist. Mit ihr lassen sich biologische Systeme, insbesondere proteinhaltige wässrige Flüssigkeiten untersuchen. Die Probe wird dabei weder verändert noch zerstört und es kann unter nativen Bedingungen des Biomoleküls gearbeitet werden, . d.h. es kann der "Ist-Zustand" gemessen werden, weil weder eine Fixierung noch ein andersartige Aufbereitung der Proben notwendig ist.

Da alle molekularen Bestandteile des Proteins Absorptionsbanden im infraroten Spektralbereich besitzen, können nahezu alle Bereiche eines Proteins beobachtet und detaillierte Informationen über die Struktur des Proteins erhalten werden.

Das MID-FTIR-Spektroskopie-Verfahren ermöglicht die automatisiert und reproduzierbar Erkennung und Quantifizierung von Änderungen der Protein-Konformation und die Bestimmung der Proteinkonzentration. Es können bereits sehr geringe Proteinkonzentrationen (bis unter 0,1 mg/ml) und geringste Konformations-Änderungen detektiert werden.

Bei der Durchführung des Verfahrens im Zuge der vorliegenden Erfindung wurden die Blutproben mit und ohne Goldpartikel im "Ist"-Zustand in einer hoch präzisen biokompatiblen und für wässrige Proben geeigneten spektroskopischen Messzelle (Transmissionszelle) analysiert. Unter Verwendung interner Kalibrationen wurde von jeder vermessenen Probe automatisch die Konzentration des gelösten Proteins und dessen Sekundärstruktur (alpha-Helix, beta-Faltblatt) bestimmt.

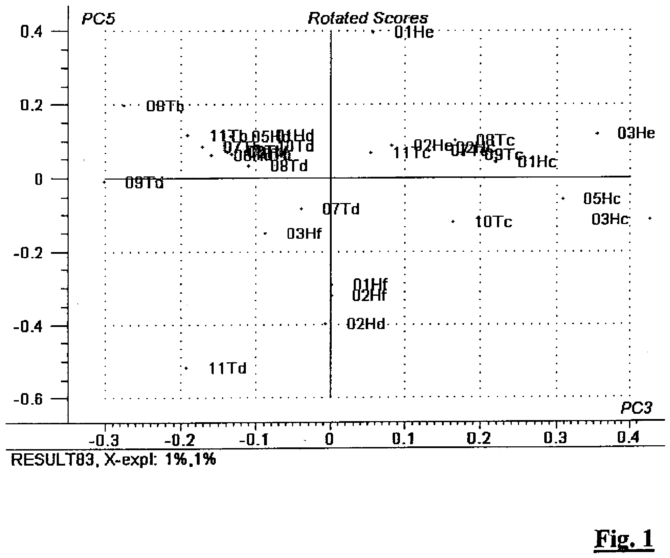

Die Ergebnisse dieser Messung sind in Fig. 1 graphisch dargestellt. Sie zeigen, dass die erfindungsgemäß in Gegenwart von Goldpartikeln inkubierten Proben sich biologisch anders verhalten als die Kontrollproben. Die Messwerte aller Proben zum Zeitpunkt T0 sind mit blauer Farbe gekennzeichnet und liegen überwiegend im linken oberen Quadranten. Die Messwerte nach Durchführung des erfindungsgemäßen Verfahrens mit einer Inkubationsdauer von 24 Stunden sind mit grüner Farbe gekennzeichnet und liegen überwiegend im oder nahe dem linken unteren Quadranten. Das zeigt an, dass die physiologische Proteinalterung im Rahmen der Blutinkubation durch den Zusatz von Gold gehemmt wird. Die Messwerte der Kontrollproben nach einer Inkubationsdauer von 24 Stunden sind mit roter Farbe gekennzeichnet und liegen überwiegend im rechten oberen Quadranten. Dieser Shift nach rechts zeigt eine Alterung der Proteinsiruktur an.

Mit einem Multiparameter-Analyseverfahren (Synonym: Multiplex-Analyse) auf der Basis von unterscheidbar codierten Mikropartikeln (z.B. dem BioPlex™2200-System der Firma BioRad Laboratories, München) wurden folgende Proteine in den Proben mit dem erfindungsgemäßen Verfahren ("Erfndung"), dem Verfahren gemäß WO 9909051 ("StdT") und entsprechenden Kontrollen ohne Behandlung zu den Zeitpunkten T0 und T24 quantitativ bestimmt:
Gelsolin (GS), Interleukin-4 (IL-4), Interleukin-10 (IL-10), Interleukin-13 (IL-13), Interleukin-1 Rezeptorantagonist (IL-1Ra), Interleukin-1ß (IL-1ß), Tumornekrosefaktor alpha (TNF-a), Granulocyte-Colony Stimulating factor (G-CSF), Granulocyte-Macrophage Colony Stimulating factor (GM-CSF), Interferon Gamma (IFN-g), Hematopoietic Stem cell growth factor (SCGF-ß), Macrophage inflammatory protein-1a (MIP-1a), Macrophage inflammatory protein-1ß (MIP-1ß), Vascular endothelial growth factor (VEGF), Interleukin-18 (IL-18), Macrophage chemotactic protein (MCP-3), Stromal derived factor (SDF-a), Fibroblast growth factor basic (FGF), Growth-regulated oncogene alpha (GROa).

Diese Proteine spielen bei der Gewebedegeneration und der Gewebereparation wichtige Rollen.

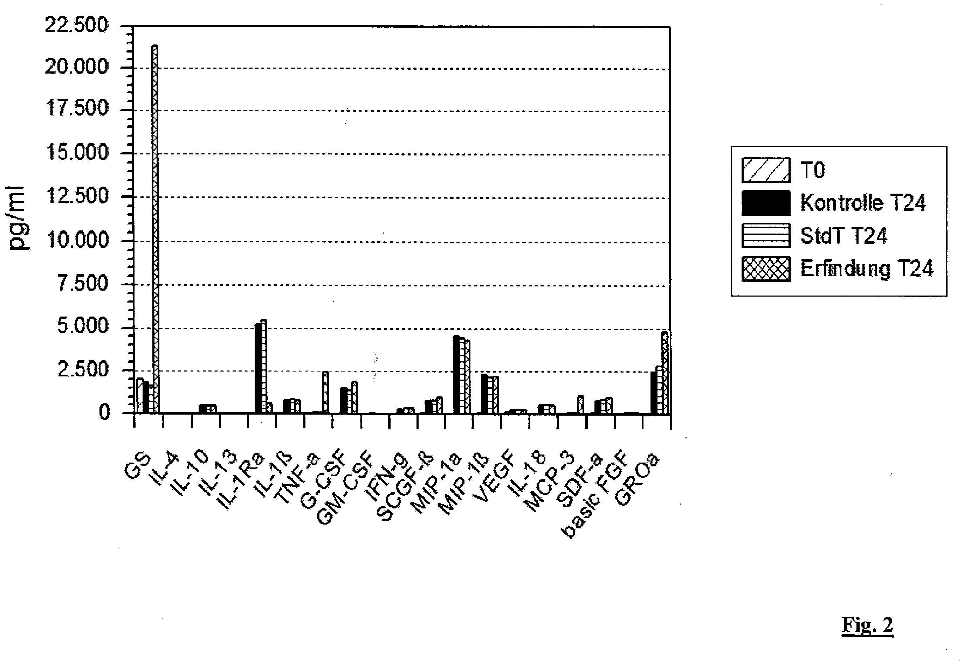

Die Ergebnisse dieser Analyse sind in Tabelle 1 und graphisch in Fig. 2 dargestellt.

Sie zeigen, dass bei den Proben ("Erfindung") nach 24 Stunden eine erhebliche Zunahme der Gelsolinkonzentration (Faktor 10) stattgefunden hat, während bei den Vergleichsproben ("Kontrolle-T24" und "StdT-T24") keine Gelsolinanreicherung sondern eher ein Gelsonlinschwund festgestellt wurde. Auch die Konzentrationen von Tumornekrosefaktor alpha (TNF-a), Macrophage chemotactic protein (MCP-3) und Growth-regulated oncogene alpha ("GROa") waren in den erfindungsgemäß behandelten Proben ("Erfindung") wesentlich (TNF-a: Faktor 30-40, MCP-3: Faktor 20-30) oder zumindest deutlich (GROa: Faktor 2) höher als in den Vergleichsproben ("Kontrolle-T24" und "StdT-T24").

### Beispiel 2: Arzneimittel-Wirksamkeitsstudien am Tier

### A) Weichteilschwellungen

Im Rahmen einer prospektiven klinischen Studie wurden 8 Pferde mit einer ausgeprägten Weichteilschwellung aufgrund von Tendinosen (degenerative Veränderungen an Sehnen im Bereich der Knochenansätze) mit dem erfindungsgemäßen Arzneimittel behandelt.

Für die Herstellung des Arzneimittels wurde ein Behältnis in Form einer Goldpartikel enthaltenden Spritze mit Blut des betreffenden Tieres gefüllt und 24 Stunden bei einer Temperatur im Bereich von 37°C inkubiert. Nach Ablauf der Inkubationszeit war das Arzneimittel in Gestalt des sich in der Spritze befindenden Blutes mit den zwischenzeitlich synthetisierten und angereicherten Proteinen, insbesondere Zytokinen und Gelsolin, sowie den Goldpartikeln fertig gestellt und konnte direkt und unmittelbar eingesetzt werden.

Da das Arzneimittel in einer Spritze hergestellt wurde, konnte es ohne Umfüllung und damit ohne die Risiken von Kontamination und Materialverlust dem betreffenden Tier per Injektion verabreicht werden.

Für die vorliegende Studie wurden zum Zeitpukt T0 für jedes Pferd vier derartige Arzneimittel-Dosen hergestellt, d.h. es wurden vier Goldpartikel enthaltende Spritzen mit Blut des betreffenden Tieres gefüllt und 24 Stunden bei einer Temperatur im Bereich von 37°C inkubiert.

Diese Arzneimittel-Injektionen wurden dem jeweiligen Pferd im Abstand von jeweils einer Woche verabreicht. Die erste Verabreichung erfolgte zum Zeitpunkt T24, die zweite, dritte und vierte nach Woche 1, nach Woche 2 und nach Woche 3. Die Arzneimittel-haltigen Spritzen für die zweite, dritte und vierte Applikation wurden bis zu ihrer Verwendung bei minus 20°C gelagert.

Der Schwellungszustand wurde nach 1 Woche, 2 Wochen, 3 Wochen, 3 Monaten, 6 Monaten und 1 Jahr überprüft und anhand einer Skala von 0-5 (0 = keinerlei Schwellung, 5 = massive Schwellung) bewertet.

In allen 8 Fällen wurde bereits nach 3 Wochen eine signifikante Reduktion der Schwellung festgestellt. Nach 6 Monaten und auch nach einem Jahr waren alle Pferde komplett schwellungsfrei. Es wurden im Rahmen der Behandlungen keinerlei Nebenwirkungen festgestellt.

Die Ergebnisse dieser Studie sind in Fig. 3 graphisch dargestellt.

### B) Lahmheiten

In einer weiteren Pferdestudie wurden 11 Pferde mit dem klinischen Krankheitssymptom der Lahmheit (12 betroffene Extremitäten) mit dem erfindungsgemäßen Arzneimittel behandelt. Die medizinischen Ursachen für die Lahmheiten waren in sechs Fällen degenerative Knorpelveränderungen (n=6) in/an Gelenken und in sechs Fällen Weichteilerkrankungen (n=6).

Für die Herstellung des Arzneimittels wurden wiederum pro Pferd vier Behältnisse in Form Goldpartikel enthaltender Spritzen mit Blut des betreffenden Tieres gefüllt und 24 Stunden bei einer Temperatur im Bereich von 37°C inkubiert. Nach Ablauf der Inkubationszeit war das Arzneimittel in Gestalt des sich in der Spritze befindenden Blutes mit den zwischenzeitlich synthetisierten und angereicherten Proteinen, insbesondere Zytokinen und Gelsolin, sowie den Goldpartikeln im Prinzip fertig gestellt. Um das Injektionsvolumen zu minimieren wurden in einem anschließenden Zentrifugationsverfahren korpuskuläre Anteile durch Zentrifugation über 10 Minuten bei 5000 U/min entfernt. Nur die jeweiligen Überstandes wurden für die Injektionen eingesetzt.

Diese Arzneimittel-Injektionen wurden dem jeweiligen Pferd im Abstand von jeweils einer Woche verabreicht. Die erste Verabreichung erfolgte zum Zeitpunkt T24, die zweite, dritte und vierte nach Woche 1, nach Woche 2 und nach Woche 3. Die Arzneimittel-haltigen Spritzen für die zweite, dritte und vierte Applikation wurden bis zu ihrer Verwendung bei minus 20°C gelagert.

Die Lahmheit wurde nach 1, 2 und 3 Wochen, 3 und 6 Monaten und 1 Jahr überprüft und der Lahmheitsgrad wurde anhand einer Skala von 0-4 (0 = keinerlei Lahmheit, 5 = massive Lahmheit) gemäß AAEP = "American Association of Equine Practitioners" bewertet.

In allen 12 Fällen wurde bereits nach 3 Wochen eine signifikante Reduktion der Lahmheit festgestellt. Nach 6 Monaten und auch nach einem Jahr waren alle Pferde komplett symptomfrei, insbesondere lahmfrei. Es wurden im Rahmen der Behandlungen keinerlei Nebenwirkungen festgestellt.

Die Ergebnisse dieser Studie sind in Fig. 4 graphisch dargestellt.

### Beispiel 3: Herstellung eines erfindungsgemäßen Behältnisses mit Goldpartikeln

Als Goldpartikel wurde Goldpuder (Partikelgröße 1 µm, Firma Bio-Rad Laboratories, München) verwendet. Die Goldpartikel wurden zunächst sterilisiert, wie vom Hersteller empfohlen: Die benötigte Menge Goldpartikel/Goldpuder, beispielsweise 30 mg, wurde mit 1 ml 70 %igen Ethanol versetzt, 10 Minuten unter leichtem Rühren (Mischer, Vortexer) inkubiert, nach 1 Minute absetzen kurz zentrifugiert und anschließend der Überstand entfernt. Danach wurden die Goldpartikel dreimal mit je 1 ml sterilem Aqua bidest. gewaschen. Nach dem letzten Waschgang mit abschließender Zentrifugation und Abschütten des Überstands wurden die Goldpartikel in sterilem PBS resuspendiert und auf die gewünschte Konzentration, z.B. 60 mg/ml), eingestellt. Unter ständigem Schütteln wurden mit einer Pipette 10µl der Goldpartikellösung entnommen und in eine S-Monovette - neutral/9ml (92 x 16 mm) von Sarstedt (REF 02.1726.001) überführt. Die Monovette wurde zunächst in einer sterilen Werkbank geöffnet (Schraubverschluss), die 10µl Goldpartikellösung auf die innere Spritzenwand appliziert und anschließend wieder verschlossen. Die befüllten Monovetten lagerten bis zur ihrer Verwendung bei Raumtemperatur. Für den Einsatz bei der Durchführung des offenbarten Verfahrens wurden in die derart vorbereiteten Monovetten 9 ml Körperflüssigkeit (z.B. Blut) aufgezogen und mit der Goldpartikel/PBS Lösung gemischt.

### Beispiel 4: Arzneimittel-Wirksamkeitsstudien am Menschen

Im Rahmen einer prospektiven Längsschnittstudie wurden durch einen zugelassenen Arzt insgesamt 9 Patienten bzw. 13 Gelenke mit röntgenologisch nachgewiesener Gonarthrose behandelt. Der Arthrosegrad war nach Kellgren-Lawrence-Klassifkation (Kellgren J.H. and Lawrence J.S.: "Radiological Assessment of Osteo-Arthrosis", Ann. rheum. Dis. (1957), 16, 494-501) mit Grad 3-4 einzustufen. Alle Patienten erhielten jeweils vier intraartikuläre Injektion mit dem offenbarungsgemässen zunächst goldbehandelten und anschließend von Partikeln befreiten autologen Serum im Anstand von einer Woche. Bei Vorliegen eines intraartikulären Ergusses wurde dieser jeweils vor der Injektion abpunktiert, die Punktionsmenge dokumentiert und zur weiteren Aufarbeitung in 1ml Aliquots bei minus 20°C eingefroren. Die Dokumentation des klinischen Behandlungsergebnisses erfolgte mit dem validierten Punktebewertungssystem (Synonym: Score) "KOOS" (Roos E.M., Roos H.P., Lohmander L.S., Ekdahl C., Beynnon B.D.: "Knee Injury and Osteoarthritis Outcome Score (KOOS - development of a selfadministered outcome measure", J. Orthop. Sports Phys. Ther. 1998, 28:88-96) vor der Behandlung, sowie 1, 3, 6 und 12 Monate nach der Behandlung. Die maximal erreichbare Punktzahl betrug 100. Je höher die Punktzahl, desto besser war das erreichte Ergebnis. Die Ergebnisse sind in Fig. 5 graphisch dargestellt.

Die Auswertung des KOOS-Score hinsichtlich der Parameter 'Symptome' und 'Sportaktivität' zeigte nach 3 und. 6 Monaten eine deutliche Verbesserung der klinischen Symptomatik (vgl. Fig.5).

Bei einer Patientin zeigte sich initial eine erhebliche Ergussbildung. Der Erguss wurde vor der Injektions-Behandlung jeweils abpunktiert, hinsichtlich der Ergussmenge dokumentiert und die Synoviapunktate hinsichtlich der Gelsolinkonzentration untersucht. Da die Gelsolinkonzentration vom Verdünnungsgrad der Ergussbildung abhängig ist, wurde die Gelsolinkonzentration auf die Urea-Konzentration bezogen (Kraus et al.: Urea as a Passive Transport Marker for Arthritis Biomarker Studies, ARTHRITIS & RHEUMATISM Vol. 46, No. 2, February 2002, pp 420-427). Die Ergebnisse sind in Fig. 6 graphisch dargestellt.

Durch die intraartikuläre Applikation des hergestellten Serums, d.h. des Bluts nach Inkubation mit Goldpartikeln und anschließender Entfernung der (aller) Partikel konnte die intraartikuläre Gelsolinkonzentration deutlich gesteigert werden (vgl. Fig.6). Gleichzeitig kam es zu einer Reduktion der Ergußmenge (vgl. Fig.6) und im weiteren Verlauf zu einer deutlichen Verbesserung der klinischen Symptomatik.

Diese Studie belegt somit die Verwendung des Arzneimittels zur Behandlung von Arthrose.

### Beispiel 5: Nachweis der chondroprotektiven Wirkung des erfindungsgemäßen Arzneimittels am in-vitro Knorpelimpaktierungsmodell

Der Einfluss mechanischer Überlastung auf den Gelenkknorpel im Rahmen der Arthroseentstehung ist hinlänglich bekannt. Es existieren auch gut etablierte und validierte in-vitro Modelle, die den Einfluss mechanischer Belastung bei Knorpel-Knochenexplantaten untersuchen, z.B. das Modell von Huser und Davies (Huser C.A. und Davies M.E.: "Validation of an in vitro single-impact load model of the initiation of osteoarthritis-like changes in articular cartilage", J Orthop Res., Apr 2006;24(4):725-732.). Ein guter Indikator für die beginnende Knorpeldegeneration in solchen Modellen ist die Messung des Proteoglykangehalt im Kulturmedium der untersuchten Knorpel-/Knochenproben.

Im Verlauf der Untersuchungen, die zu der vorliegenden Erfindung geführt haben, wurde in einer tierexperimentellen Studie am Minischwein "Göttinger Minipig" bei 6 Tieren mit 9 ml Spritzen, die jeweils 10³ Goldpartikel enthielten, pro Tier jeweils 4 Proben (à 9 ml) Blut entnommen und gemäß dem erfindungsgemäßen Verfahren 24 h bei 37°C inkubiert. Im Anschluß an die Inkubationszeit wurden die Blutproben in den Spritzenzylindern zentrifugiert und je Spritze (bzw. Spritzenzylinder) der Überstand durch einen Sterilfilter in einen frischen/neuen goldpartikelfreien Spritzenzylinder überführt.

Parallel zur Entnahme der Blutproben wurden den der betreffenden Tieren unter aseptischen Bedingungen Knorpel-Knochen-Proben aus den jeweiligen Hüftköpfen entnommen und zu 8x8x10 mm (Länge/Breite/Höhe) Blöcken zugeschnitten. Der Knorpel war bei allen Explantaten makroskopisch intakt.

Bis zum Einsatz in dem Stoßbelastungstest (Impaktierungstest) wurden Knorpel-Knochen-Probenblöcke in DMEM-Medium mit dem Zusatz von 10 % Humanem Serum (HS), 100 U/ml Pencillin, 100 µg/ml Gentamycin und 1,25 U/ml Amphotericin B aufbewahrt.

Noch am Tage der Probenentnahme wurde der Stoßbelastungstest (Impaktierungstest) durchgeführt. Dazu wurde jeweils ein Knorpel-Knochen-Probenblock/Explantat in einem zylinderförmigen Freifallturm ("drop tower") aus Polymethylmethacrylate mit den Maßen 33 cm Höhe und 4 cm axial mit Abstand unterhalb eines Stoßkolbens unter sterilen Bedingungen angeordnet. Der Stoßkolben hatte die Form eines Zylinders mit den Maßen 5 cm Höhe und 3,94 cm Durchmesser, und sein Gewicht betrug 493 g. Der Stoßkolben war über ein Gewinde mit der eigentlichen Stoßscheibe ("Impaktorstück") verbunden, die ein Gewicht von 7 g, eine Höhe/Dicke von 1 cm und einen Durchmesser von 0,6 cm aufwies. Die Stoßbelastung/Impaktierung erfolgte einmalig pro Knorpel-Knochen-Probenblock im "drop tower" durch den freien Fall des Stoßkolbens mit Stoßscheibe (Impaktors) aus 15 cm Höhe unter auf die Probe. Die Stoßbelastung betrug dabei 0.736 J auf eine Knorpeloberfläche von 28.3 mm².

Die Knorpel-Knochen-Probenblöcke/Explantate wurden in 3 Gruppen unterteilt:
- Explantate ohne Stoßbehandlung = "nicht-impaktierte Kontrollen" = "0-Kontrollen")
- Explantate mit Stoßbehandlung = "impaktierte Kontrollen" = "Stoß-Kontrollen"
- Explantate mit Stoßbehandlung und anschließender Inkubation mit dem erfindungsgemäß hergestellten, proteinangereichtem Blutserum

Im Anschluß an die Stoßbehandlung wurden die behandelten Knochen-Knorpel-Präparate und ebenso die 0-Kontrollen 3 mal mit PBS gewaschen und in 12-well Kulturplatten überführt. Jedes Explantat wurde mit 3 ml des jeweiligen Behandlungsmediums angesetzt und unter standardisierten Bedingungen (37°C, 5 % CO₂,) im Brutschrank inkubiert. Das Kulturmedium wurde alle 72 Stunden gewechselt.

Das offenbarungsgemäss hergestellte proteinangereichte Blutserum wurde bei den Explantat der betreffenden Explantat-Gruppe am Tag 0 und Tag 7 dem zugesetzt.

Am Tag 2, 7 und 14 wurde bei allen Testansätzen der Proteoglykangehalt im Kulturmedium gemessen. Hierzu wurde der Blyscan Gylcosaminoglycan Assay der Firma Biocolor Ltd., (Carrickfergus, UK) verwendet. Die Ergebnisse sind in der Graphik von Fig. 7 dargestellt. Der Proteoglykangehalt ist als mittlere Gylcosaminoglycan (GAG)-Konzentration in µg/ml Medium angegeben.

Die Analyse der Proteoglykanfreisetzung zeigt, dass in allen drei Gruppen, nämlich den 0-Kontrollen und den beiden stoßbehandelten/impaktierten Explantaten/ Knorpel-Knochen-Präparaten die Proteogykanmenge mit der Zeit ansteigt.

Bei den mit dem erfindungsgemäß hergestellten proteinangereichten Blutserum behandelten Explantaten/Knorpel-Knochen-Präparaten ist dieser Anstieg jedoch nur wenig stärker als bei den nicht-impaktierten Kontrollen (0-Kontrollen), während die stoßbehandelten/impaktierten Explantate (Knorpel-Knochen-Präparate) ohne entsprechende Serumbehandlung einen sehr viel höheren Anstieg zeigen.

Dieser Test belegt somit den chondroprotektiven Effekt des erfindungsgemäßen Arzneimittels.

**Tabelle 1: Multiparameter-Analyseverfahren**

| **Protein** | **T0** | **Kontrolle T24** | **StdT T24** | **Erf T24** |
|---|---|---|---|---|
| **GS** | 1990 | 1798 | 1634 | 21260 |
| **IL-4** | 1 | 6 | 7 | 6 |
| **IL-10** | 5 | 444 | 438 | 447 |
| **IL-13** | 2 | 2 | 2 | 2 |
| **IL-1Ra** | 1 | 5130 | 5387 | 603 |
| **IL-1ß** | 1 | 808 | 854 | 798 |
| **TNF-a** | 4 | 78 | 56 | 2444 |
| **G-CSF** | 6 | 1454 | 1367 | 1890 |
| **GM-CSF** | 10 | 53 | 45 | 49 |
| **IFN-g** | 34 | 302 | 312 | 320 |
| **SCGF-ß** | 64 | 800 | 800 | 1000 |
| **MIP-1a** | 1 | 4500 | 4400 | 4300 |
| **MIP-1ß** | 84 | 2330 | 2100 | 2150 |
| **VEGF** | 154 | 286 | 290 | 282 |
| **IL-18** | 27 | 524 | 520 | 540 |
| **MCP-3** | 1 | 56 | 80 | 1019 |
| **SDF-a** | 64 | 780 | 852 | 993 |
| **basic FGF** | 31 | 73 | 79 | 62 |
| **GROa** | 40 | 2460 | 2800 | 4800 |

## Patentansprüche

1. Zytokine- und Gelsolin angereichertes Blut zur Verwendung als Arzneimittel, erhältlich dadurch, dass Blut in ein Goldpartikel enthaltendes Behältnis aufgenommen wird und dass diese Mischung aus Blut und Goldpartikeln inkubiert wird.

2. Blut zur Verwendung nach Anspruch 1, **dadurch gekennzeichnet, dass** inkubiert wird und anschließend die Goldpartikel aus dem Blut entfernt und verworfen werden.

3. Blut zur Verwendung nach Anspruch 1, **dadurch gekennzeichnet, dass** Goldpartikel und Blutzellen und/oder andere unlöslichen Bestandteile aus dem Blut entfernt und verworfen werden.

4. Blut zur Verwendung nach einem der Ansprüche 1 bis 3, **dadurch gekennzeichnet, dass** die Goldpartikel eine Größe von etwa 1 µm aufweisen.

5. Blut zur Verwendung nach einem der Ansprüche 1 bis 4, **dadurch gekennzeichnet, dass** die Goldpartikel in einer Menge von 10³ - 10⁴ Goldpartikel pro 10 ml Behältnis oder von 0.3 mg Goldpartikel mit einem Durchmesser 1 µm pro 1 ml Blut/Behältnis vorliegen.

6. Blut nach einem der Ansprüche 1 bis 5, zur Verwendung in der Behandlung von Arthrose.

## Claims

1. Cytokine- and gelsolin-enriched blood for use as a medicament, obtainable by collecting blood in a container that contains gold particles, and incubating said mixture of blood and gold particles.

2. Blood for use according to claim 1, **characterized in that** after incubation the gold particles are removed from the blood and discarded.

3. Blood for use according to claim 1, **characterized in that** gold particles and blood cells and/or other insoluble components are removed from the blood and discarded.

4. Blood for use according to any one of claims 1 to 3 , **characterized in that** the gold particles have a size of about 1 µm.

5. Blood for use according to any one of claims 1 to 4, **characterized in that** the gold particles are present in an amount of 10³ - 10⁴ gold particles per 10 ml container or in an amount of 0,3 mg gold particles with a diameter of 1 µm per 1 ml of blood/container.

6. Blood according to any one of claims 1 to 5, for use in the treatment of osteoarthrosis.

## Revendications

1. Sang enrichi en cytokines et en gelsolines pour une utilisation en tant que médicament, pouvant être obtenu du fait que le sang est collecté dans un récipient contenant des particules d'or et du fait que ce mélange à base de sang et de particules d'or est incubé.

2. Sang pour une utilisation selon la revendication 1, **caractérisé en ce que** l'incubation est réalisée puis les particules d'or sont éliminées du sang et écartées.

3. Sang pour une utilisation selon la revendication 1, **caractérisé en ce que** les particules d'or et les cellules sanguines et/ou d'autres constituants non solubles sont éliminés du sang et écartés.

4. Sang pour une utilisation selon l'une des revendications 1 à 3, **caractérisé en ce que** les particules d'or présentent une taille d'environ 1 µm.

5. Sang pour une utilisation selon l'une des revendications 1 à 4, **caractérisé en ce que** les particules d'or sont présentes en une quantité de 10³ à 10⁴ particules d'or par récipient de 10 ml ou de 0,3 mg de particules d'or de diamètre de 1 µm par 1 ml de sang/récipient.

6. Sang selon l'une des revendications 1 à 5 pour une utilisation dans le traitement de l'arthrose.
